# EUROPEAN PATENT APPLICATION

(11) **EP 1 721 906 A1**
(43) Date of publication of application: **15.11.2006**
(21) Application number: 05719869.9
(22) Date of filing: 03.03.2005
(51) Int. Cl.: C07D 501/56, A61K 31/546, A61P 31/04

(54) **3-PYRIDINIUM METHYLCEPHEM COMPOUND**

(30) Priority: 05.03.2004 JP 2004061506
(71) Applicant: Shionogi Co., Ltd., Osaka-shi, Osaka 541-0045 (JP)
(72) Inventor: Nishitani, Yasuhiro, c/o Shionogi & Co., Ltd., Osaka-shi, Osaka 5530002 (JP); Yasukata, Tatsuro, c/o Shionogi & Co., Ltd., Osaka-shi, Osaka 5530002 (JP); Yamawaki, Kenji, c/o Shionogi & Co., Ltd., Osaka-shi, Osaka 5530002 (JP)
(74) Representative: Vossius & Partner
(86) International application number: PCT/JP2005/003565
(87) International publication number: WO 2005/085258

(57) **Abstract**

A compound of Formula 1: 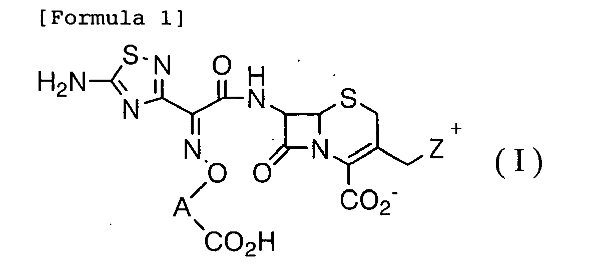 (wherein A is optionally substituted lower alkylene (substituent: mono- or di- lower alkyl, lower alkylidene, or lower alkylene having two or more carbons); Z⁺ is either of the groups shown below: 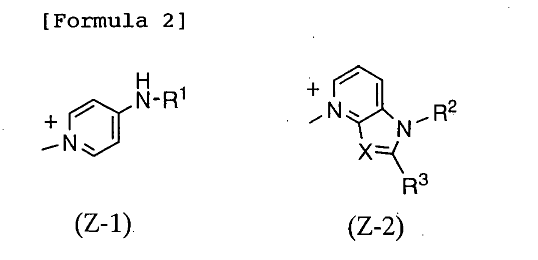 (wherein R¹ and R² are each independently hydrogen, optionally substituted amino lower alkyl or optionally substituted cyclic amino; R³ is hydrogen or amino; X is N or CR⁴ (R⁴ is hydrogen or optionally substituted lower alkyl)), a pharmaceutically acceptable salt or a solvate thereof.

## Description

### [Technical field]

The present invention relates to broad spectrum cephem compounds having broad antibacterial spectrum over various pathogenic bacteria and to pharmaceutical compositions containing the same.

### [Background art]

Study of so-called broad spectrum cephem compounds having potent antibacterial activities against various Gram-positive and Gram-negative bacteria including Pseudomonas aeruginosa has recently been focused on compounds in which 7-terminal of cephem backbone is substituted with aminothiazole or aminothiadiazole and3-position with a cyclic-type quarternary ammoniummethyl group. For example, a cephem compound having an imidazopyridiniummethyl group at its 3-side chain is known (see Patent document 1). In particular, as a cephem compound which is effective against Gram-negative bacteria such as Pseudomonas aeruginosa, compounds having a carboxy-substituted alkoxyimino structure at its 7-side chain are known (see Patent document 2, 3). Patent document 2 discloses cephem compounds in which 7-position is a substituted aminothiazole type and having various quaternary ammonium methyl groups at 3-position. Patent document 3 discloses cephem compounds in which 7-position is aminothiadiazole and a hetero cycle at 3-position is a pyrazole ring. Additionally, compounds having a carboxy-substituted alkoxyimino structure at its 7-side chain are known (see Patent document 4, 5).
[Patent document 1] WO 00/32606
[Patent document 2] WO 03/07440
[Patent document 3] WO 02/090364
[Patent document 4] JP-A 57-131795
[Patent document 5] JP-A 60-231684

### [Disclosure of the invention]

### [Problems to be solved by the invention]

It has been desired to develop novel broad spectrum cephem compounds having more potent antibacterial activities or exhibiting different antibacterial patterns compared to conventional cephem compounds.

### [Means for solving the problem]

The present inventors have intensively studied to find out that a cephem compound having an aminothiadiazole ring and a carboxy-substituted alkoxyimino structure at its 7-side chain, and a substituted pyridinium ring as a hetero ring on its 3-side chain exhibits excellent antibacterial activity, and have accomplished the present invention shown below.
(1) a compound of Formula 1: 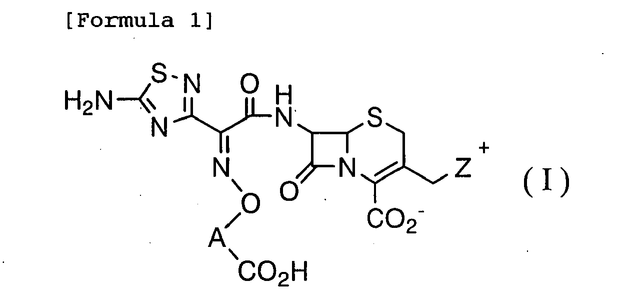 (wherein A is optionally substituted lower alkylene (substituent: mono- or di- lower alkyl, lower alkylidene, or lower alkylene having two or more carbons); Z⁺ is either of the groups shown below: 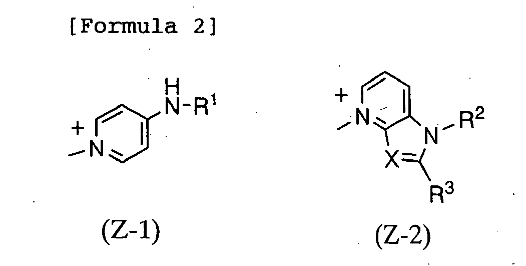 (wherein R¹ and R² are each independently hydrogen, optionally substituted amino lower alkyl or optionally substituted cyclic amino; R³ is hydrogen or amino; X is N or CR⁴ (R⁴ is hydrogen or optionally substituted lower alkyl)), a pharmaceutically acceptable salt or a solvate thereof.
(2) a compound according to the above (1), wherein A is a group of Formula 3: 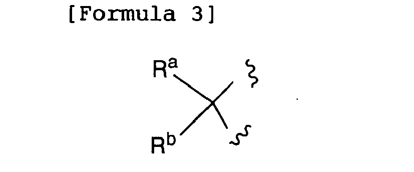 (wherein R^{a} and R^{b} are each independently hydrogen or lower alkyl, or taken together form lower alkylidene or lower alkylene having two or more carbons), a pharmaceutically acceptable salt or a solvate thereof.
(3) a compound according to the above (1), wherein A is -C(CH3)₂-, a pharmaceutically acceptable salt or a solvate thereof.
(4) a compound according to the above (1), wherein R¹ and R² are each independently hydrogen, amino lower alkyl which may be substituted with lower alkyl or saturated 4- to 6-membered cyclic amino which may be substituted with lower alkyl; R³ is hydrogen or amino; and X is N or CR⁴(R⁴ is hydrogen, lower alkyl, or lower alkyl substituted with amino which may be substituted with lower alkyl), a pharmaceutically acceptable salt or a solvate thereof.
(5) a compound according to the above (1), wherein R¹ and R² are each independently amino lower alkyl which may be substituted with lower alkyl, or saturated 4- to 6-membered cyclic amino which may be substituted with lower alkyl; R³ is amino; and X is N or CH, a pharmaceutically acceptable salt or a solvate thereof.
(6) a compound according to the above (1), wherein A is a group of Formula: -C(CH₃)₂-; R¹ and R² are each independently amino lower alkyl which may be substituted with lower alkyl or saturated 4- to 6-membered cyclic amino which may be substituted with lower alkyl; R³ is hydrogen or amino; and X is N or CR⁴(R⁴ is hydrogen, lower alkyl, or lower alkyl substituted with amino which may be substituted with lower alkyl), a pharmaceutically acceptable salt or a solvate thereof.
(7) a compound according to the above (1), wherein Z⁺ is a group of (Z-1); and R¹ is amino lower alkyl which may be substituted with lower alkyl, a pharmaceutically acceptable salt or a solvate thereof.
(8) a compound according to the above (1), wherein A is a group of Formula: -C(CH₃)₂-; Z⁺ is a group of (Z-1); and R¹ is lower alkyl substituted with amino which may be substituted with lower alkyl, a pharmaceutically acceptable salt or a solvate thereof.
(9) a compound according to the above (1), wherein A is a group of Formula: -C(CH₃)₂-; Z⁺ is a group of (Z-1); and R¹ is -(CH₂)mNHCH₃ (m is an integer of 1 to 5), a pharmaceutically acceptable salt or a solvate thereof.
(10) a compound according to the above (1), wherein A is a group of Formula: -C(CH₃)₂-; Z⁺ is a group of (Z-1); and R¹ is -(CH₂)₂NHCH₃, a pharmaceutically acceptable salt or a solvate thereof.
(11) a compound according to the above (1), wherein A is a group of Formula: -C(CH₃)₂-; Z⁺ is a group of (Z-1); and R¹ is saturated 4- to 6-membered cyclic amino which may be substituted with lower alkyl, a pharmaceutically acceptable salt or a solvate thereof.
(12) a compound according to the above (1), wherein Z⁺ is a group of (Z-2); and R³ is amino, a pharmaceutically acceptable salt or a solvate thereof.
(13) a compound according to the above (1), wherein A is a group of Formula: -C(CH₃)₂-; Z⁺ is a group of (Z-2); and R³ is amino, a pharmaceutically acceptable salt or a solvate thereof.
(14) a compound according to the above (1), wherein A is a group of Formula: -C(CH₃)₂-; Z⁺ is a group of (Z-2); and R² is hydrogen, -(CH₂)nNHCH₃ (n is an integer of 1 to 5), -(CH₂)pCH(CH₃)NH₂ (p is an integer of 1 to 5) or either one of the groups shown below: 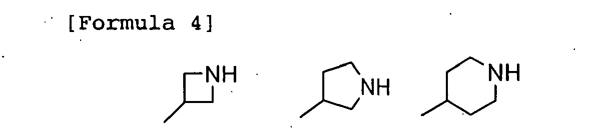 ; R³ is hydrogen or amino; and X is N or CR⁴(R⁴ is hydrogen, lower alkyl or lower alkyl substituted with amino which may be substituted with lower alkyl), a pharmaceutically acceptable salt or a solvate thereof.
(15) a compound according to the above (1), wherein A is a group of Formula: -C(CH₃)₂-; Z⁺ is a group of (Z-2); R² is -(CH₂)nNHCH₃ (n is an integer of 1 to 5); R³ is amino; and X is N, a pharmaceutically acceptable salt or a solvate thereof.
(16) a compound according to the above (1), wherein A is a group of Formula: -C(CH₃)₂-; Z⁺ is a group of (Z-2); R² is -(CH₂)₃NHCH₃; R³ is amino; and X is N, a pharmaceutically acceptable salt or a solvate thereof.
(17) a compound according to the above (1), wherein A is a group of Formula: -C(CH₃)₂-; Z⁺ is a group of (Z-2); R² is saturated 4-to 6-membered cyclic amino which may be substituted with lower alkyl; R³ is hydrogen or amino; and X is N or CR⁴(R⁴ is hydrogen, or lower alkyl), a pharmaceutically acceptable salt or a solvate thereof.
(18) a compound according to the above (1), wherein A is a group of Formula: -C(CH₃)₂-; Z⁺ is a group of (Z-2) ; R² is saturated 4-to 6 -membered cyclic amino which may be substituted with lower alkyl; R³ is amino; and X is N or CH, a pharmaceutically acceptable salt or a solvate thereof.
(19) a compound according to the above (1), wherein A is a group of Formula: -C(CH₃)₂-; Z⁺ is a group of (Z-2); R² is hydrogen; R³ is hydrogen or amino; and X is CR⁴ (R⁴ is lower alkyl or lower alkyl substituted with amino which may be substituted with lower alkyl), a pharmaceutically acceptable salt or a solvate thereof.
(20) a pharmaceutical composition containing a compound according to any one of the above (1) to (19), a pharmaceutically acceptable salt or a solvate thereof.
(21) a pharmaceutical composition of the above (20), which is an antibacterial agent.
(22) a carboxy and/or amino-protected compound of a compound of any one of the above (1) to (19), a pharmaceutically acceptable salt or a solvate thereof.

### [Effect of the invention]

Compounds of the present invention exhibit potent antibacterial activities against various kinds of bacteria. Preferred compounds have outstandingly potent antibacterial activities against Gram-negative bacteria including Escherichia coli and resistive Pseudomonas aeruginosa, in particular. Other preferred compounds show balanced activities against Gram-positive and Gram-negative bacteria. Further, compounds of the present invention are especially suited for injectable agents because they are superior in disposition and water-solubility.

### [Best mode for carrying out the invention]

Unless otherwise mentioned, each term, by itself or as part of another, has a common meaning.
Examples of lower alkyl include a straight or branched C1 to C6 alkyl such as methyl, ethyl, n-propyl, i-propyl, t-butyl, n-pentyl, and n-hexyl.
Lower alkylene means methylene or a binary group which is generated when two hydrogen atoms are lost from two different carbon atoms in a lower alkyl, and is preferably represented by -(CH₂)ₘ₋(m is an integer of 1 to 6, preferably 1 to 3).
Lower alkylidene means a binary group which is generated when two hydrogen atoms are lost from a single carbon atom in a lower alkyl, and examples include =CH₂, =CHCH₃, =CHCH₂CH₃, =C(CH₃)₂, =CH(CH₂)₂CH₃, and =CHC(CH₃)₃.
A is not particularly limited insofar as it is a binary group that will not exert an adverse affect on antibacterial activity and disposition of Compound (I), and is preferably optionally substituted lower alkylene, more preferably optionally substituted methylene. Examples of substituent include mono- or di- lower alkyl (e.g., mono on dimethyl), lower alkylidene (e.g.,=CH₂), or lower alkylene having two or more carbon atoms (e.g., -(CH₂)m-(m: 2 or 3)), with mono-ordi- lower alkyl being preferred, and dilower alkyl (e.g., dimethyl) being more preferred.
A is more preferably a group of Formula 5: 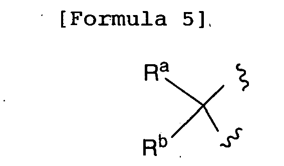 (wherein R^{a} and R^{b} are each independently hydrogen or lower alkyl, or taken together form a lower alkylidene or lower alkylene having two or more carbons).
R^{a} and R^{b} both are preferably lower alkyl, and more preferably C1-C3 alkyl, and particularly preferably methyl. That is, A is particularly preferably "=C(CH₃)₂". When R^{a} and R^{b} taken together form lower alkylene having two or more carbons, they form a 3- or more-membered carbon ring (e.g., cyclopropane, cyclobutane) together with an adjacent carbon atom.
Z⁺ is a group shown by (Z-1) or (Z-2).
R¹ and R² are each independently hydrogen, optionally substituted amino lower alkyl or optionally substituted cyclic amino.
Examples of substituent of "optionally substituted amino lower alkyl" include lower alkyl (e.g., methyl, ethyl, propyl), with methyl being particularly preferred.
When R¹ is "optionally substituted amino lower alkyl", it is preferably a group represented by -(CH₂)mNHCH₃ (m is an integer of 1 to 5, preferably an integer of 1 to 3, particularly preferably 2).
When R² is "optionally substituted amino lower alkyl", it is preferably a group represented by -(CH₂)nNHCH₃ (n is an integer of 1 to 5, preferably 1 to 3, particularly preferably 3), or a group represented by - (CH₂)pCH(CH₃)NH₂ (p is an integer of 1 to 5, preferably an integer of 1 to 3, particularly preferably 2).
Examples of substituent of "optionally substituted cyclic amino " include lower alkyl (e.g., methyl, ethyl, propyl), with methyl being particularly preferred.
"Cyclic amino" encompasses saturated, unsaturated or aromatic homocyclic (e.g., 4- to 7-membered) or condensed cyclic (e.g., 8- to 10-membered) amino, which may further include one to three identical or different hetero atom(s) selected from O,S and N beside the N atom of amino which is a constituting atom of the ring. "Cyclic amino" is preferably a saturated and/or homocyclic (preferably 4- to 6-membered) amino. More preferably, it is azetidyl, pyrrolidyl, piperidyl or the like. Binding position of cyclic amino is preferably 3- or 4-position with respect to the amino. More preferred examples of cyclic amino are as follows. 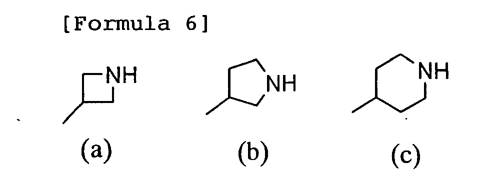 When R¹ is cyclic amino, it is preferably pyrrolidyl (more preferably (b)).
When R² is cyclic amino, it is preferably piperidyl (more preferably (c)).
R³ is hydrogen or amino, and preferably amino.
X is N or CR⁴ (R⁴ is hydrogen or optionally substituted lower alkyl), and preferably N. When X is CR⁴, examples of substituent for "optionally substituted lower alkyl" include amino which may be substituted with lower alkyl, hydroxy and the like, with amino (e.g., NH₂, NHCH₃) which may be substituted with lower alkyl being preferred. R⁴ is preferably hydrogen, lower alkyl (e.g., methyl), lower alkyl amino lower alkyl (e.g., -CH₂NHCH₃, -CH₂CH₂NHCH₃) and the like, and more preferably hydrogen or lower alkyl (e.g., methyl).
Examples of preferred embodiments of Compound (I) are shown below.
(1) Where R¹ and R² are each independently hydrogen, amino lower alkyl which may be substituted with lower alkyl or saturated 4- to 6-membered cyclic amino which may be substituted with lower alkyl; R³ is hydrogen or amino; and X is N or CR⁴(R⁴ is hydrogen, lower alkyl or lower alkyl substituted with amino which may be substituted with lower alkyl).
(2) Where R¹ and R² are each independently amino lower alkyl which may be substituted with lower alkyl, or saturated 4- to 6-membered cyclic amino which may be substituted with lower alkyl; R³ is amino; and X is N or CH.
(3) Where A is a group of Formula: -C(CH₃)₂-; R¹ and R² are each independently amino lower alkyl which may be substituted with lower alkyl or saturated 4- to 6-membered cyclic amino which may be substituted with lower alkyl; R³ is hydrogen or amino; X is N or CR⁴(R⁴ is hydrogen, lower alkyl or lower alkyl substituted with amino which may be substituted with lower alkyl).
(4) When Z⁺ is a group shown by (Z-1), R¹ is preferably amino lower alkyl which may be substituted with lower alkyl, and more preferably A is a group of Formula: -C(CH₃)₂-. More preferably, R¹ is -(CH₂)mNHCH₃ (m is an integer of 1 to 5), and particularly preferably, m is 2.
(5) When Z⁺ is a group shown by (Z-1), A is preferably a group of Formula: -C(CH₃)₂-; and R¹ is saturated 4- to 6-membered cyclic amino which may be substituted with lower alkyl.
(6) When Z⁺ is a group shown by (Z-2), R³ is preferably amino, and more preferably, A is a group of Formula: -C(CH₃)₂-.
(7) When Z⁺ is a group shown by (Z-2), A is a group of Formula: -C(CH₃)₂-; R² is hydrogen, -(CH₂)nNHCH₃ (n is an integer of 1 to 5), -(CH₂)pCH(CH₃)NH₂ (p is an integer of 1 to 5) or either one of the groups shown below: 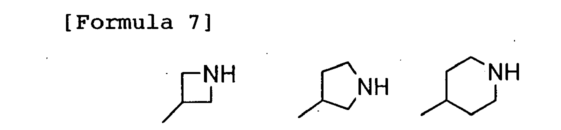 ; R³ is hydrogen or amino; X is N or CR⁴(R⁴ is hydrogen, lower alkyl or lower alkyl substituted with amino which may be substituted with lower alkyl). More preferably, R² is - (CH₂)nNHCH₃ (n is an integer of 1 to 5); R³ is amino; and X is N. Particularly preferably, R² is -(CH₂)₃NHCH₃; R³ is amino; and X is N.
(8) When Z⁺ is a group shown by (Z-2), A is a group of Formula: -C(CH₃)₂-; and R² is saturated 4- to 6-membered cyclic amino which may be substituted with lower alkyl. Preferably, R³ is hydrogen or amino; and X is N or CR⁴ (R⁴ is hydrogen, or lower alkyl). More preferably, R³ is amino; and X is N or CH.
(9) When Z⁺ is a group shown by (Z-2), A is a group of Formula: -C(CH₃)2-; R² is hydrogen; R³ is hydrogen or amino; and X is CR⁴ (R⁴ is lower alkyl or lower alkyl substituted with amino which may be substituted with lower alkyl).

Examples of preparation method of compounds of the present invention are shown below. 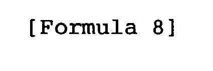 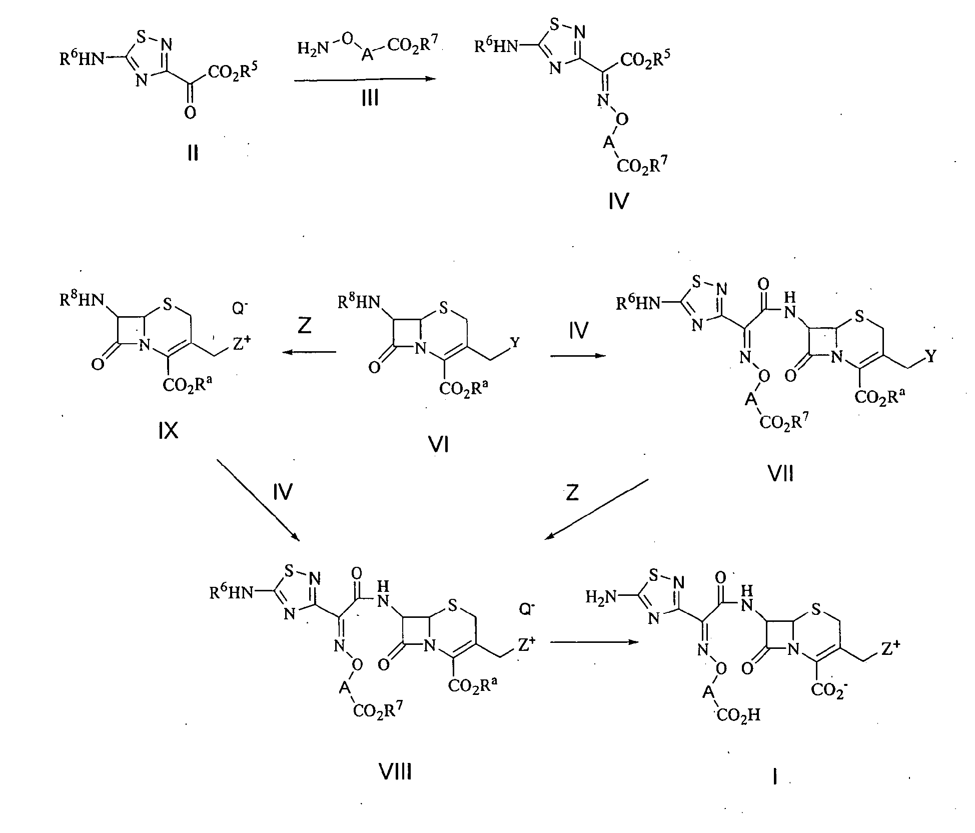 (wherein R⁵ is hydrogen or carboxy protecting group; R⁶ is hydrogen or amino protecting group; R⁷ is hydrogen or carboxy protecting group; R⁸ is hydrogen or amino protecting group; R^{a} is hydrogen or carboxy protecting group; Y is a leaving group (e.g., hydroxy, halogen (Cl, Br, I or the like), carbamoyloxy, substituted carbamoyloxy, acyloxy, methanesulfonyloxy, toluenesulfonyloxy or the like); Q⁻ is a counter ion of halogen or the like)
(1) Method of preparing Compound (IV)
   Compound (IV) is a known compound as described in, for example, WO02/090364 or obtained by reaction between Compound (II) and Compound (III). In this case, preferably R⁵ is hydrogen; R⁶ is amino protecting group; -R⁷ is carboxy protecting group.
   Use amount of Compound (III) is usually about 1 to 10 mols, preferably about 1 to 2 mols, per 1 mol of Compound (II).
   Examples of solvents used in the reaction include ethers (e.g., dioxane, tetrahydrofuran, diethylether, tert-butylmethylether, and diisopropylether), esters (e.g., ethyl formate, ethyl acetate, and n-butyl acetate), halogenated hydrocarbons (e.g., dichloromethane, chloroform, and carbon tetrachloride), hydrocarbons (e.g., n-hexane, benzene, and toluene), alcohols (e.g., methanol, ethanol, isopropanol), amides (e.g., formamide, N,N-dimethylformamide, N,N-dimethylacetamide, and N-methylpyrrolidone), ketones (e.g., acetone and methylethylketone), nitryls (e.g., MeCN and propionitryl), dimethylsulfoxide, and water. These solvents may be used singly or in combination of two or more kinds.
   Reaction temperature is usually about -20 to 100° C, preferably about 0 to 50°C.
(2) Method of preparing Compounds (VII) and (VIII)
   1) Acylation at 7-position
      By reaction between Compound (VI) and Compound (IV), Compound (VII) is obtained. In this case, preferably R^{a} is carboxy protecting group; R⁵ is hydrogen; R⁶ is amino protecting group; R⁷ is carboxy protecting group; and R⁸ is hydrogen.
      Use amount of Compound (IV) is usually about 1 to 5 mols, preferably about 1 to 2 mols, per 1 mol of Compound (VI).
      Examples of solvents used in the reaction include ethers (e.g., dioxane, tetrahydrofuran, diethylether, tert-butylmethylether, and diisopropylether), esters (e.g., ethyl formate, ethyl acetate, and n-butyl acetate), halogenated hydrocarbons (e.g., dichloromethane, chloroform, and carbon tetrachloride), hydrocarbons (e.g., n-hexane, benzene, and toluene), amides (e.g., formamide, N,N-dimethylformamide, N,N-dimethylacetamide, and N-methylpyrrolidone), ketones (e.g., acetone and methylethylketone), nitryls (e.g., MeCN and propionitryl), dimethylsulfoxide, and water.
      Reaction temperature is usually about -40 to 100° C, preferably about 0 to 30° C.
      Compound (VI, VII, VIII, T=SO) may also be obtained by oxidizing Compounds (VI,VII,VIII,T=S). Preferably, Compound (VII, T=SO) is obtained by oxidizing Compound (VII,T=S).
      Examples of the oxidant include m-Cl perbenzoic acid (m-CPBA), hydrogen peroxide, and peracetic acid.
      Compound (VI) may be synthesized according to the method described in documents (e.g., JP-A 60-231684, JP-A 62-149682 and the like).
      The above amidation may be conducted after converting the carboxyl moiety to a reactive derivative (e.g. , inorganic base salt, organic base salt, acid halide, acid azide, acid anhydride, mixed acid anhydride, active amide, active ester, and active thioester). The inorganic base includes alkaline metals (e.g., Na and K) and alkaline earth metals (e.g., Ca and Mg); the organic base includes trimethylamine, triethylamine, tert-butyldimethylamine, dibenzylmethylamine, benzyldimethylamine, N-methylmorpholine and diisopropylethylamine; the acid halide includes acid chloride and acid bromide; the mixed acid anhydride includes mixed monoalkylcarboxylic acid anhydride, mixed aliphatic carboxylic acid anhydride, mixed aromatic carboxylic acid anhydride, mixed organic sulfonic acid anhydride; and the active amide includes amide formed with heterocyclic compound containing N atom, for example. Examples of the active ester include organic phosphate esters (e.g., diethoxy phosphate ester and diphenoxy phosphate ester), p-nitrophenyl ester, 2,4-dinitrophenyl ester, cyanomethyl ester. The active thioester includes esters formed with aromatic heterocyclicthiol compound (e.g., 2-pyridylthiol ester). The above reaction may be carried out using an appropriate condensing agent, if necessary. Examples of the condensing agent include, for example, 1-dimethylaminopropyl-3-ethylcarbodiimide hydrochloride (WSCD·HCl), N,N'-dicyclohexylcarbodiimide, N,N'-carbonyldiimidazole, N,N'-thiocarbonyldiimidazole, N-ethoxycarbonyl-2-ethoxy-1,2-dihydroquinoline, phosphorus oxychloride, alkoxyacetylene, 2-chloropyridiniummethyl iodide, 2-fluoropyridiniummethyl iodide, and trifluoroacetic anhydride.
   2) Formation of 3-side chain
      By reaction between Compound (VII) and Z (heterocyclic compound corresponding to Z⁺), Compound (VIII) is obtained. In this case, preferably R⁶ is amino protecting group; R⁷ is carboxy protecting group; and R^{a} is carboxy protecting group. When a functional group such as amino is present as a substituent on Z of Compound (VIII), it may be protected by a protecting group.
      Use amount of Z is usually about 1 to 10 mols, preferably about 1 to 2 mols, per 1 mol of Compound (VII).
      Examples of solvents used in the reaction include ethers (e.g., dioxane, tetrahydrofuran, diethylether, tert-butylmethylether, and diisopropylether), esters (e.g., ethyl formate, ethyl acetate, and n-butyl acetate), halogenated hydrocarbons (e.g. , dichloromethane, chloroform, and carbon tetrachloride), hydrocarbons (e.g., n-hexane, benzene, and toluene), amides (e.g., formamide, N,N-dimethylformamide, N,N-dimethylacetamide, and N-methylpyrrolidone),ketones(e.g.,acetone and methylethylketone), nitryls (e.g., MeCN and propionitryl), dimethylsulfoxide, and water.
      Reaction temperature is usually about 0 to 100° C, preferably about 0 to 50°C, more preferably about 10 to 30°C.
      As a reaction promoter, NaI, KI or the like may be used.
(3) Method of preparing Compounds (IX) and (VIII)
   1) Formation of 3-side chain
      By reaction between Compound (VI) and Z, Compound (IX) is obtained. In this case, preferably R⁸ is hydrogen; and R^{a} is carboxy protecting group. When a functional group such as amino is present as a substituent on Z of Compound (IX), it may be protected by a protecting group.
      Use amount of Z is usually about 1 to 10 mols, preferably about 1 to 2 mols, per 1 mol of Compound (VI).
      Examples of solvents used in the reaction include ethers (e.g., dioxane, tetrahydrofuran, diethylether, tert-butylmethylether, and diisopropylether), esters (e.g., ethyl formate, ethyl acetate, and n-butyl acetate), halogenated hydrocarbons (e.g., dichloromethane, chloroform, and carbon tetrachloride), hydrocarbons (e.g., n-hexane, benzene, and toluene), amides (e.g., formamide, N,N-dimethylformamide, N,N-dimethylacetamide, and N-methylpyrrolidone), ketones (e.g., acetone and methyl ethyl ketone), nitryls (e.g., MeCN and propionitryl), dimethyl sulfoxide, and water.
      Reaction temperature is usually about 0 to 100° C, preferably about 0 to 50°C, more preferably about 10 to 30°C.
      As a reaction promoter, NaI, KI or the like may be used.
   2) Acylation at 7-position
      By reaction between Compound (IX) and Compound (IV), Compound (VIII) is obtained. In this case, preferably R^{a} is carboxy protecting group; R⁵ is hydrogen; R⁶ is amino protecting group; R⁷ is carboxy protecting group; and R⁸ is hydrogen.
      Use amount of Compound (IV) is usually about 1 to 5 mols, preferably about 1 to 2 mols, per 1 mol of Compound (IX).
      Examples of solvents used in the reaction include ethers (e.g., dioxane, tetrahydrofuran, diethylether, tert-butylmethylether, and diisopropylether), esters (e.g., ethyl formate, ethyl acetate, and n-butyl acetate), halogenated hydrocarbons (e.g., dichloromethane, chloroform, and carbon tetrachloride), hydrocarbons (e.g., n-hexane, benzene, and toluene), amides (e.g., formamide, N,N-dimethylformamide, N,N-dimethylacetamide, and N-methylpyrrolidone), ketones (e.g., acetone and methylethylketone), nitryls (e.g., MeCN and propionitryl), dimethylsulfoxide, and water.
      Reaction temperature is usually about -40 to 100°C, and preferably about 0 to 30°C.
      Amidation may be achieved by converting a carboxyl moiety to a reactive derivative (e.g., inorganic basic salt, organic basic salt, acid halide, acid azide, acid anhydride, mixed acid anhydride, active amide, active ester, active thioester) as is the same with the above, and an appropriate condensing agent may be used as necessary.
(4) Deprotection
   By subjecting Compound (VIII) to deprotection according to a technique well-known in the art, Compound (I) is obtained.
   Examples of solvents used in the reaction include ethers (e.g., anisole, dioxane, tetrahydrofuran, diethylether, tert-butylmethylether, and diisopropylether), esters (e.g., ethyl formate, ethyl acetate, and n-butyl acetate), halogenated hydrocarbons (e.g., dichloromethane, chloroform, and carbon tetrachloride), hydrocarbons (e.g. , n-hexane, benzene, and toluene), amides (e.g., formamide, N,N-dimethylformamide, N,N-dimethylacetamide, and N-methylpyrrolidone), ketones (e.g., acetone and methylethylketone), nitryls (e.g., MeCN and propionitryl), nitros (e.g., nitromethane, nitroethane, nitrobenzene), dimethylsulfoxide, and water. These solvents may be used singly or in combination of two or more kinds.
   Reaction temperature is usually about -70 to 50 °C, preferably about -50 to 0°C.
   As a catalyst, Lewis acids (e.g., AlCl₃, SnCl₄, and TiCl4), protonic acids (e.g., HCl, H₂SO₄, HClO₄, HCOOH, phenol) and the like may be used, and anisole may be used as well if necessary.
   Obtained Compound (I) may further be chemically modified to synthesize other form of Compound (I), a pharmaceutically acceptable salt or solvate thereof.
   The present invention also provides a carboxy and/or amino protected compound of Compound (I), a pharmaceutically acceptable salt or solvate thereof. Concretely, above Compound (VIII) is exemplified. This compound is useful as a synthesis intermediate.
   Carboxy protected compound of Compound (I) means a compound in which preferably, a carboxyl on 7-side chain has an ester structure (COOR⁷) and/or a carboxy at 4-position has an ester structure (COOR^{a}). These esters embrace esters that are readily metabolized in a body to become carboxyl states.
   Examples of the carboxy protecting group shown by R⁷ or R^{a} include lower alkyls (e.g., methyl, ethyl, t-butyl), (substituted) aralkyls (e.g., benzyl, benzhydryl, p-methoxybenzyl, p-nitrobenzyl) and silyl groups (t-butyldimethylsilyl, diphenyl t-butylsilyl).
   Amino protected compound of Compound (I) means a compound in which amino on 3-side chain and/or aminothiadiazole ring at 7-position is protected (e.g., NHR⁶). Amino protecting group shown by R⁶ embraces groups that are readily metabolized in a body to become amino, and examples of which include lower alkoxycarbonyl (e.g., t-butoxycarbonyl, benzyloxycarbonyl, p-nitrobenzyloxycarbonyl), (substituted)aralkanoyl (e.g., p-nitorobenzoyl), and acyl (e.g., formyl, chloroacetyl).
   The above carboxy and/or amino protected compound may be deprotected according to the method described in the preparation method (4).
   Examples of the pharmaceutically acceptable salt of Compound (I) include salts formed with inorganic bases, ammonia, organic bases, inorganic acids, organic acids, basic amino acids, halogen ions or the like, and inner salts. Examples of the inorganic base include alkali metal (e.g., Na and K) and alkaline earth metal (e.g., Mg), and examples of the organic base include procaine, 2-phenylethylbenzylamine, dibenzylethylenediamine, ethanolamine, diethanolamine, tris(hydroxymethyl)aminomethane, polyhydroxyalkylamine, and N-methyl glucosamine. Examples of the inorganic acid include hydrochloric acid, hydrobromic acid, sulfuric acid, nitric acid, and phosphoric acid. Examples of the organic acid include p-toluene sulfonic acid, methanesulfonic acid, formic acid, trifluoroacetic acid and maleic acid. Examples of the basic amino acid include lysine, algin, ornithine and histidine.
   As a solvent for solvate of Compound (I), water and alcohol are exemplified.

Compounds of the present invention have broad spectrum antibacterial activities, and can be used for prophylaxis or therapy of various diseases caused by pathogenic bacteria in various mammalian including human being, such as air duct infection, urinary tract infection, respiratory tract infection, septicaemia, nephritis, cholecystitis, intraoral infection, endocarditis, pneumonia, meningitidis, middle otitis, enteritis, maxillary empyema, wound infection, opportunistic infection and the like.
Compounds of the present invention have particularly high antibacterial activities against Gram-negative bacteria including Pseudomonas aeruginosa, Escherichia coli and Haemophilus influenzae, Inaddition, since they are stable against B-lactamase (inparticular, type C β-lactamase) produced by cephem resistant Pseudomonas aeruginosa, they are effective for resistant Pseudomonas aeruginosa. Therefore', excellent therapeutic effect is exerted by single application rather than using β-lactamase inhibitor as well. Further, compounds of the present invention also have antibacterial activities against Gram-positive bacteria including Staphylococcus aureus, penicillin-resistant Staphylococcus pneumoniae (PRSP) and pneumococcus. Particularly preferred compounds exhibit high antibacterial activities against Gram-negative bacteria such as Pseudomonas aeruginosa, and exhibit balanced antibacterial activities against Gram-positive bacteria. They also exhibit properties of disposition, such as higher blood concentration, extended duration of efficacy, and significant transition to tissues.
Compounds of the present invention can be parenterally or orally administered in a form of injection, capsule, tablet, granule or the like, and a preferred form is injection because of high water-solubility of the compounds. The daily dosage can usually be varied in the range of about 0.1 to 100 mg, preferably about 0.5 to 50 mg, per 1 kg of body weight of patient or animal, which is administered in two to four divisions if necessary. Carriers used for injections include e. g. , distilled water, brine, and pH adjusting agents such as bases. Carries for preparing capsules, granules, and tablets can be excipients known in the art (e.g. , starch, lactose, sucrose, calcium carbonate, calcium phosphate), binders (e.g., starch, Arabian gum, carboxymethyl cellulose, hydroxypropyl cellulose, crystalline cellulose), and lubricants (e.g., magnesium stearate, talc).
Examples are shown below.
(Abbreviation)
Me=methyl; Et=ethyl; iPr=isopropyl; Bu^{t}=t-butyl; Ac=acetyl;
DMF=dimethylformamide; THF=tetrahydrofuran;
Boc=t-butoxycarbonyl; PMB=p-methoxybenzyl; BH=benzhydryl

### [Example 1]

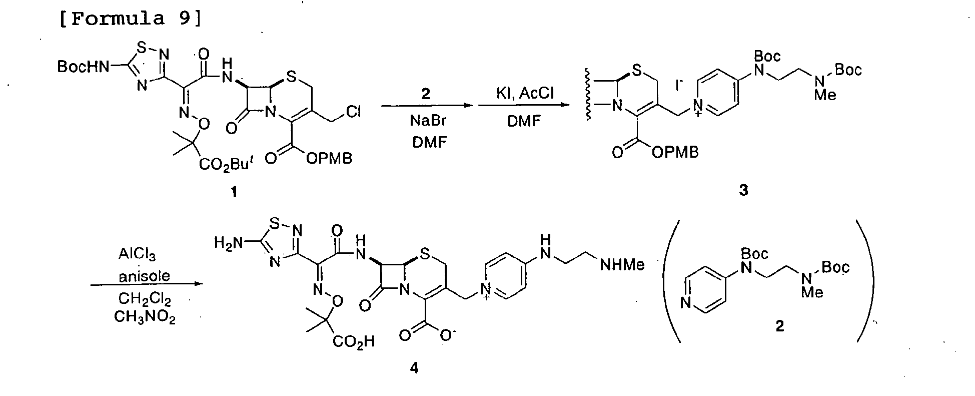

(1) Under nitrogen atmosphere, a solution of Compound 2 (425 mg, 1.2 mmol) in dimethylformamide (2.6 mL) was added with Compound 1 (877mg, 1.1mmol) and sodium bromide (229 mg, 2.2 mmol), and stirred for 12 hours at room temperature. To this reaction solution, were added dimethylformamide (8 mL) and potassium iodide (1.28 g, 7.7 mmol), and after cooling to -40°C, acetyl chloride (0.31 mL, 4.4 mmol) was added dropwise. After raising the temperature to 0°C, the mixture was stirred for an hour under ice-cooling, and the resultant reaction solution was added dropwise to 5% brine' (100 mL) supplemented with sodium thiosulfate (1.5 g) under ice-cooling, to give precipitates. After stirring for another 30 minutes under ice-cooling, the precipitates were collected by filtration, dried over phosphoric chloride under reduced pressure, to give a crude product containing 3 in the form of ochre powder (1.33 g). The crude product was dissolved in a mixture of methylene chloride (13 mL) and nitromethane (13 mL), added with anisole (1.41 mL, 12.9 mmol) under nitrogen atmosphere and cooled to -40° C. After adding dropwise aluminum chloride (2.0M nitromethane solution, 6.5 mL, 12.9 mmol) under -40°C, the solution was stirred for an hour at 0°C. This reaction solution was poured into a mixture of 1N hydrochloric acid aqueous solution (30 mL) and diethylether (80mL) that was stirred under ice-cooling, and after separation of an aqueous phase, anorganic phase was extracted twice with 0.5N hydrochloric acid aqueous solution (30 mL). After concentrating combined aqueous phases under reduced pressure, fractions collected through HP chromatography were lyophilized, to give Compound 4 (colorless powder, 568 mg).
   ¹H-NMR (D₂O) d: 1.50 (3H, s), 1.51 (3H, s), 2.76 (3H,s), 3.17 and 3.59 (2H, ABq, J = 17.9 Hz), 3.22 (2H, t, J = 6.3Hz), 3.72 (2H, t, J = 6.3 Hz), 4.83 and 5.10 (2H, ABq, J = 14.7 Hz), 5.25 (1H, d, J = 4.8 Hz), 5.81 (1H, d, J = 4.8 Hz), 6.89 (2H, d, J = 7.5 Hz), 8.19 (2H, brd-like).
   IR (KBr) cm⁻¹: 3387, 3066, 1771, 1650, 1598, 1557, 1523, 1467, 1398, 1361, 1289, 1217, 1167, 1065.
   MS(ESI): 620⁺ (M+H)⁺
   Elementary Analysis C₂₆H₂₉N₁₁O₇S₂·3.4 H₂O
   Calc.: C,42.61 ; H,4.92 ; N,21.02 ; S,8.75 (%)
   Found: C,42.67 ; H,4.68 ; N,21.05 ; S,8.55 (%)

### [Example 2]

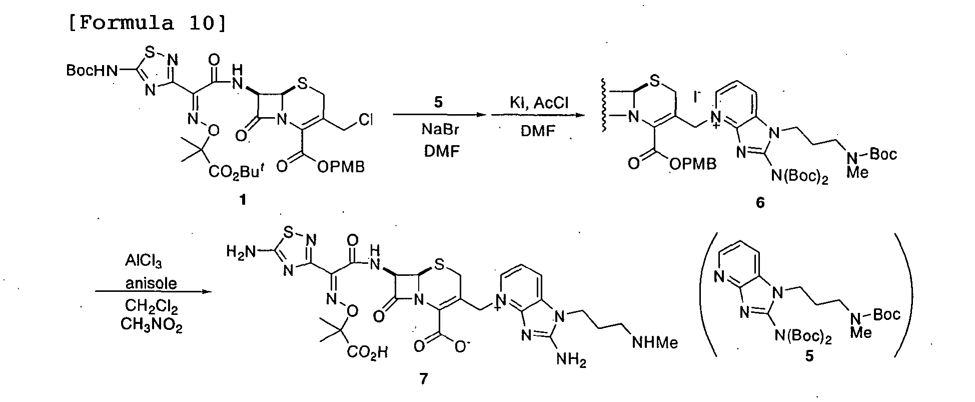

(1) Under nitrogen atmosphere, a solution of Compound 5 (389 mg, 0.77 mmol) in dimethylformamide (1.7 mL) was added with Compound 1 (558 mg, 0.7 mmol) and sodium bromide (144 mg, 1.40 mmol), and stirred for 12 hours at room temperature. To this reaction solution, were added dimethylformamide (6 mL) and KI (813 mg, 4.90 mmol), and after cooling to -40° C, acetyl chloride (0.20 mL, 2.8 mmol) was added dropwise. After raising the temperature to 0°C, the mixture was stirred for an hour under ice-cooling, and the resultant reaction solution was added dropwise to 5% brine (100 mL) supplemented with sodium thiosulfate (1.0 g) under ice-cooling, to give precipitates. After stirring for another 30 minutes under ice-cooling, the precipitates were collected by filtration, dried over phosphoric chloride under reduced pressure, to give a crude product containing 6 in the form of ochre powder (944mg). The crude product was dissolved in a mixture of methylene chloride (10 mL) and nitromethane (10 mL), added with anisole (0.88 mL, 8.13 mmol) under nitrogen atmosphere and cooled to -40° C. After adding dropwise aluminum chloride (2.0M soln. in CH₃NO₂, 4.1mL, 8.2 mmol) under -40° C, the solution was stirred for an hour at 0° C. This reaction solution was poured into a mixture of 1N hydrochloric acid aqueous solution (30 mL) and diethylether (80mL) that was stirred under ice-cooling, and after separation of an aqueous phase, an organic phase was extracted twice with 0.5N hydrochloric acid aqueous solution (30 mL). After concentrating combined aqueous phases under reduced pressure, fractions collected through HP chromatography were lyophilized, to give Compound 7 (colorless powder, 360 mg).
¹H-NMR (D₂O) d: 1.49 (3H, s), 1.50 (3H, s), 2.15 - 2.26 (2H, m), 2.71 (3H, s), 3.10 - 3.15 (2H, m), 3.26 and 3.51 (2H, ABq, J = 18.0 Hz), 4.23 (2H, t-like), 5.20 (1H, d, J = 4.8 Hz), 5.24 and 5.57 (2H, ABq, J = 15.0 Hz), 5.83 (1H, d, J = 4.8 Hz), 7.29 (1H, dd, J = 6.8, 7.7 Hz), 7.88 (1H, dd, J = 0.7, 7.7), 8.11 (1H, dd, J = 0.7, 6.8).
IR (KBr) cm⁻¹: 3377, 1770, 1651, 1601, 1566, 1495, 1469, 1398, 1362, 1320, 1223, 1162, 1067.
MS(ESI): 674⁺ (M+H)⁺

| Elementary Analysis C₂₆H₃₁N₁₁O₇S₂·4.2 H₂O | | | | |
|---|---|---|---|---|
| Calc.: | C,41.67 ; | H,5.30 ; | N,20.56 ; | S,8.56 (%) |
| Found: | C,41.68 ; | H,5.16 ; | N,20.46 ; | S,8.67 (%) |

The followng compounds were synthesized according to Example 1 or 2.

### [Example 3]

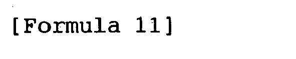

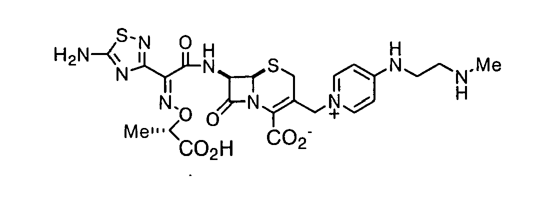

¹H-NMR (D₂O) d: 1.47 (3H, d, J = 6.9 Hz), 2.75 (3H, s), 3.17 and 3.58 (2H, ABq, J = 17.9 Hz), 3.32 (2H, t, J = 6.0Hz), 3.73 (2H, t, J = 6.0 Hz), 4.73 (1H, q, J = 6.9 Hz), 4.88 and 5.08 (2H, ABq, J = 14.7 Hz), 5.24 (1H, d, J = 4.8Hz), 5.55 (1H, d, J = 4.8 Hz), 6.91 (2H, d, J = 7.8 Hz), 8.18 (2H, brd-like).
IR (KBr) cm⁻¹: 3396, 3065, 1771, 1650, 1601, 1557, 1523, 1453, 1397, 1358, 1287, 1218, 1169, 1095, 1065, 1035.
MS(ESI): 606⁺ (M+H)⁺

| Elementary Analysis C₂₃H₂₇N₉O₇S₂·3.6 H₂O | | | | |
|---|---|---|---|---|
| Calc.: | C,41.20 ; | H,5.14 ; | N,18.80 ; | S,9.56 (%) |
| Found: | C,41.16 ; | H,4.74 ; | N,18.79 ; | S,9.13 (%) |

### [Example 4]

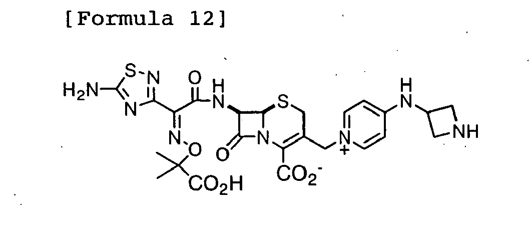

¹H-NMR (D₂O) d: 1.60 (6H, s), 3.29 and 3.66 (2H, ABq, J = 18.3 Hz), 4.20 - 4.27 (2H, m), 4.50 - 4.57 (2H, m), 4.18 (1H, m), 4.99 and 5.34 (2H, ABq, J = 15.0 Hz), 5.31 (1H, d, J = 5.1 Hz), 5.91 (1H, d, J = 5.1 Hz), 6.91 (2H, brd-like), 8.22 (2H, brd, J = 7.8 Hz).
IR (KBr) cm⁻¹: 3398, 3060, 1769, 1649, 1601, 1553, 1468, 1400, 1362, 1291, 1217, 1166, 1065.
MS(ESI): 618⁺ (M+H)⁺

| Elementary Analysis C₂₄H₂₇N₉O₇S₂·4.5 H₂O | | | | |
|---|---|---|---|---|
| Calc.: | C,41.25 ; | H,5.19 ; | N,18.04 ; | S, 9.18 (%) |
| Found: | C,41.13 ; | H,4.91 ; | N,18.06 ; | S,9.19 (%) |

### [Example 5]

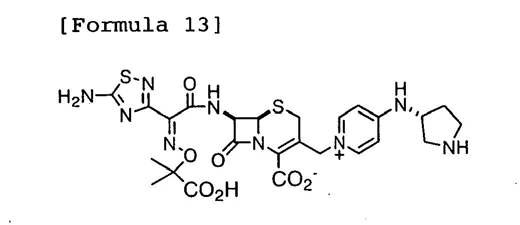

¹H-NMR (D₂O) d: 1.50 (3H, s), 1.51 (3H, s), 2.09 - 2.20 (1H, m), 2.42 - 2.54 (1H, m), 3.17 and 3.59 (2H, ABq, J = 17.7 Hz), 3.37 (1H, dd, J = 4.2, 12.9 Hz), 3.48 - 3.55 (2H, m), 3.70 (1H, dd, J = 6.6, 12.9 Hz), 4.45 - 4.52 (1H, m), 4.83 and 5.11 (2H, ABq, J = 15.0 Hz), 5.25 (1H, d, J = 4.8 Hz), 5.81 (1H, d, J = 4.8 Hz), 6.89 (2H, d, J = 7.8 Hz), 8.18 (2H, brs).
IR (KBr) cm⁻¹: 3399, 2986, 1772, 1650, 1600, 1553, 1467, 1396, 1361, 1288, 1217, 1166, 1065.
MS(ESI): 632⁺ (M+H)⁺

| Elementary Analysis C₂₅H₂₉N₉O₇S₂·3.9 H₂O | | | | |
|---|---|---|---|---|
| Calc.: | C,42.78 ; | H,5.28 ; | N,17.96 ; | S,9.14 (%) |
| Found: | C,42.53 ; | H,4.99 ; | N,18.04 ; | S,9.23 (%) |

### [Example 6]

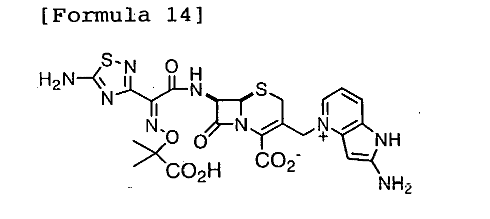

¹H-NMR (d₆-DMSO) d: 1.43 (3H, s), 1.44 (3H, s), 3.00 and 3.31 (2H, ABq, J = 17.7 Hz), 5.04 (1H, d, J = 4.8 Hz), 5.21 and 5.33 (2H, ABq, J = 14.1 Hz), 5.73 (1H, dd, J = 4.8, 8.56 Hz), 5.91 (1H, s), 6.86 (1H, t-like), 7.48 (1H, d, J = 7.5 Hz), 7.82 (2H, brs), 8.15 (1H, d, J = 6.6 Hz), 8.18 (2H, brs), 9.65 (1H, brs), 12.8 (1H, brs).
IR (KBr) cm⁻¹: 3345, 3198, 2938, 1773, 1638, 1581, 1525, 1473, 1427, 1396, 1364, 1284, 1160, 1061.
MS(ESI) : 602⁺ (M+H)⁺

| Elementary Analysis C₂₃H₂₃N₉O₇S₂·3.1 H₂O | | | | |
|---|---|---|---|---|
| Calc.: | C,42.02 ; | H,4.48 ; | N,19.17 ; | S,9.75 (%) |
| Found: | C,42.37 ; | H,4.33 ; | N,18.74 ; | S,9.82 (%) |

### [Example 7]

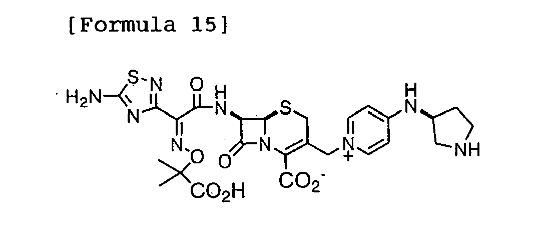

¹H-NMR (D₂O) d: 1.49 (3H, s), 1.50 (3H, s), 2.61 - 2.70 (2H, m), 3.26 and 3.51 (2H, ABq, J = 17.7 Hz), 3.51 - 3.61 (1H, m), 3.67 - 3.95 (3H, m), 5.20 (1H, d, J = 4.8 Hz), 5.24 and 5.60 (2H, ABq, J = 14.7 Hz), 5.22 - 5.35 (1H, m), 5.82 (1H, d, J = 4.8 Hz), 5.82 (1H, d, J = 4.8 Hz), 7.29 (1H, dd, J = 6.8, 7.8 Hz), 7.98 (1H, d, J = 7.8 Hz), 8.14 (1H, d, J = 6.8 Hz).
IR (KBr) cm⁻¹: 3361, 1770, 1666, 1601, 1559, 1491, 1439, 1398, 1362, 1322, 1221, 1165, 1112, 1066.
MS(ESI): 672⁺ (M+H)⁺

| Elementary Analysis C₂₆H₂₉N₁₁O₇S₂·3.4 H₂O | | | | |
|---|---|---|---|---|
| Calc.: | C,42.61 ; | H,4.92 ; | N,21.02 ; | S,8.75 (%) |
| Found: | C,42.44 ; | H,4.83 ; | N,20.97 ; | S,9.16 (%) |

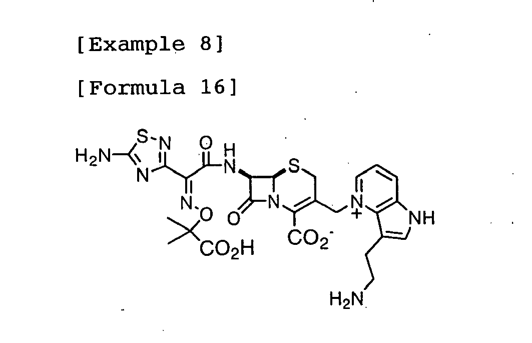

¹H-NMR (D₂O) d: 1.49 (3H, s), 1.51 (3H, s), 3.11 and 3.47 (2H, ABq, J = 18.0 Hz), 3.35- 3.42 (4H, m), 5.28 (1H, d, J = 4.5 Hz), 5.88 (1H, d, J = 4.5 Hz), 7.64 (1H, t, J = 7.2 Hz), 8.03 (1H, brs), 8.50 (2H, d, J = 7.2 Hz). IR (KBr) cm⁻¹: 3398, 1771, 1660, 1528, 1448, 1396, 1360, 1288, 1226, 1157, 1119, 1066.
MS(ESI): 630⁺ (M+H)⁺

| Elementary Analysis C₂₅H₂₇N₉O₇S₂·3.4 H₂O | | | | |
|---|---|---|---|---|
| Calc.: | C,43.46 ; | H,4.93 ; | N,18.25 ; | S,9.28 (%) |
| Found: | C,43.37 ; | H,4.67 ; | N,18.41 ; | S,9.20 (%) |

### [Example 9]

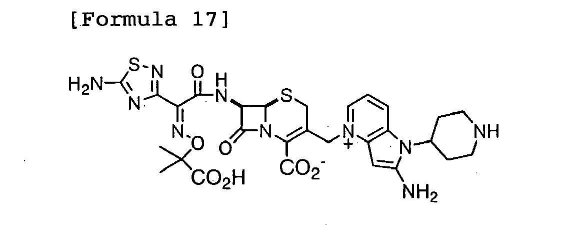

¹H-NMR (D₂O ⁺ DCl) d: 1.58 (3H, s), 1.59 (3H, s), 2.26 (2H, d, J = 13.2 Hz), 2.60 - 2.75 (2H, m), 3.21 and 3.47 (2H, ABq, J = 18.3 Hz), 3.24 - 3.34 (1H, m), 3.72 (2H, d, J = 13.2 Hz), 4.66 - 4.78 (1H, m), 5.29 (1H, d, J = 4.8 Hz), 5.34 and 5.53 (2H, ABq, J = 15.6 Hz), 5.90 (1H, d, J = 4.8 Hz), 7.07 (1H, dd, J = 6.6, 7.8 Hz), 7.93 (2H, t-like).
IR (KBr) cm⁻¹: 3382, 3183, 1770, 1594, 1559 1469, 1434, 1399, 1360, 1283, 1160, 1065.
MS(ESI): 685⁺ (M+H)⁺

| Elementary Analysis C₂₈H₃₁N₁₀O₇S₂·4.2 H₂O | | | | |
|---|---|---|---|---|
| Calc.: | C,44.23 ; | H,5.36 ; | N,18.42 ; | S,8.43 (%) |
| Found: | C,44.16 ; | H,5.08 ; | N,18.69 ; | S,7.88 (%) |

### [Example 10]

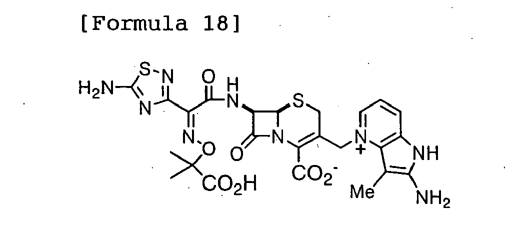

¹H-NMR (d₆-DMSO) d: 1.43 (3H, s), 1.44 (3H, s), 2.23 (3H, s), 2.98 and 3.53 (2H, ABq, J = 17.7 Hz), 5.10 (1H, d, J = 4.8 Hz), 5.39 (2H, t-like), 5.76 (1H, dd, J = 4.8, 8.1 Hz), 6.78 (1H, t-like, J = 7.2 Hz), 7.35 (1H, d, J = 7.2 Hz), 7.61 (2H, brs), 8.18 (3H, m), 9.64 (1H, brs), 12.7 (1H, brs). IR (KBr) cm⁻¹: 3351, 3197, 1773, 1635, 1567, 1523, 1477, 1397, 1364, 1284, 1224, 1162, 1092, 1060, 1016.
MS(ESI): 616⁺ (M+H)⁺

| Elementary Analysis C₂₄H₂₅N₉O₇S₂·3.5 H₂O | | | | |
|---|---|---|---|---|
| Calc.: | C,42.47 ; | H,4.75 ; | N,18.57 ; | S,9.45 (%) |
| Found: | C, 42.50 ; | H,4.18 ; | N,18.31 ; | S,9.36 (%) |

### [Example 11]

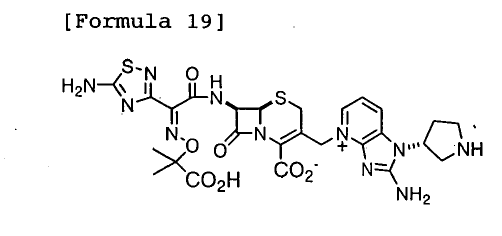

¹H-NMR (D₂O ⁺ DCl) d: 1.60 (6H, s), 2.68 (2H, q, J = 8.7 Hz), 3.35 (1H, d, J = 18.6 Hz), 3.53 - 3.63 (2H, m), 3.70 - 3.97 (3H, m), 5.27 - 5.40 (2H, m), 5.49 and 5.70 (2H, ABq, J = 14.9 Hz), 5.91 (1H, d, J = 5.1 Hz), 7.33 (1H, t-like), 8.05 (1H, dd, J = 7.8 Hz), 8.16 (1H, d, J = 6.6 Hz).
IR (KBr) cm⁻¹: 3374, 1769, 1666, 1601, 1560, 1491, 1440, 1400, 1362, 1322, 1222, 1165, 1113, 1092, 1066.
MS(ESI): 672⁺ (M+H)⁺

| Elementary Analysis C₂₆H₂₉N₁₁O₇S₂·3.4 H₂O | | | | |
|---|---|---|---|---|
| Calc.: | C,42.61 ; | H,4.92 ; | N,21.02 ; | S,8.75 (%) |
| Found: | C,42.67 ; | H,4.68 ; | N,21.05 ; | S,8.55 (%) |

### [Example 12]

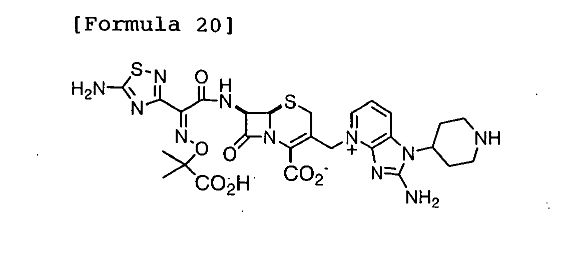

¹H-NMR (D₂O ⁺ DCl) d: 1.49 (3H, s), 1.50 (3H, s), 2.31 (2H, d, J = 12.5 Hz), 2.49 - 2.62 (2H, m), 3.19 - 3.29 (3H, m), 3.50 (1H, d, J = 17.1 Hz), 3.70 (2H, d, J = 12.5 Hz), 5.20 (1H, d, J = 4.8 Hz), 5.24 and 5.59 (2H, ABq, J = 14.7 Hz), 5.83 (1H, d, J = 4.8 Hz), 7.28 (1H, t-like), 8.03 (1H, d, J = 8.1 Hz), 8.12 (1H, d, J = 6.6 Hz).
IR (KBr) cm⁻¹: 3374, 3179, 1771, 1602, 1557, 1491, 1438, 1397, 1362, 1320, 1224, 1165, 1091, 1066, 1000.
MS(ESI): 686⁺ (M+H)⁺

| Elementary Analysis C₂₇H₃₁N₁₁S₂·4.0 H₂O | | | | |
|---|---|---|---|---|
| Calc.: | C,42.79 ; | H,5.19 ; | N,20.33 ; | S,8.46 (%) |
| Found: | C,42.49 ; | H,5.38 ; | N,20.62 ; | S,8.51 (%) |

### [Example 13]

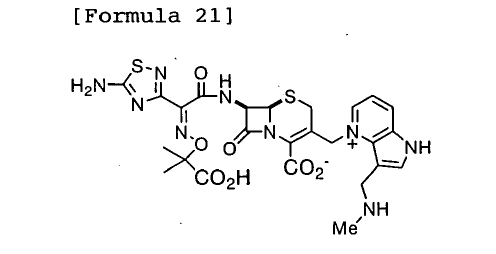

¹H-NMR (D₂O) d: 1.51 (3H, s), 1.52 (3H, s), 2.82 (3H, s), 3.13 and 3.57 (2H, ABq, J = 18.0 Hz), 5.31 (1H, d, J = 4.8 Hz), 5.72 and 5.85 (2H, ABq, J = 14.7 Hz), 5.89 (1H, d, J = 4.8 Hz), 7.72 (1H, t-like), 8.31 (1H, s), 8.54 (1H, d, J = 6.0 Hz), 8.59 (1H, d, J = 8.1 Hz).
IR (KBr) cm⁻¹: 3397, 1771, 1609, 1528, 1450, 1398, 1363, 1291, 1157, 1066.
MS(ESI): 630⁺ (M+H)⁺

| Elementary Analysis C₂₅H₂₇N₉O₇S₂·7.1 H₂O | | | | |
|---|---|---|---|---|
| Calc.: | C,39.64 ; | H,5.48 ; | N,16.64 ; | S,8.47 (%) |
| Found: | C,39.73 ; | H,4.81 ; | N,16.66 ; | S,9.28 (%) |

### [Example 14]

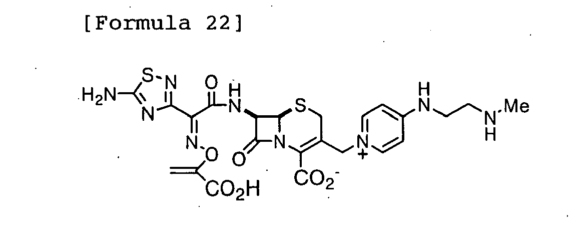

¹H-NMR (D₂O) d: 2.76 (3H, s), 3.15 and 3.55 (2H, ABq, J = 17.9 Hz), 3.33 (2H, t, J = 6.0 Hz), 3.73 (2H, t, J = 6.0 Hz), 4.86 and 5.08 (2H, ABq, J = 14.4 Hz), 5.21 - 5.23 (2H, m), 5.37 (1H, d, J = 2.1 Hz), 5.84 (1H, d, J = 4.8 Hz), 6.90 (2H, d, J = 7.5 Hz), 8.16 (2H, brd-like).
IR (KBr) cm⁻¹: 3398, 3064, 1770, 1650, 1600, 1557, 1523, 1455, 1397, 1357, 1290, 1201, 1170, 1065, 1065, 1020.
MS(ESI): 604⁺ (M+H)⁺

| Elementary Analysis C₂₃H₂₅N₉O₇S₂·3.9 H₂O | | | | |
|---|---|---|---|---|
| Calc.: | C,40.99 ; | H,4.91 ; | N,18.71 ; | S,9.52 (%) |
| Found: | C,41.05 ; | H,4.94 ; | N,18.51 ; | S,9.48 (%) |

### [Example 15]

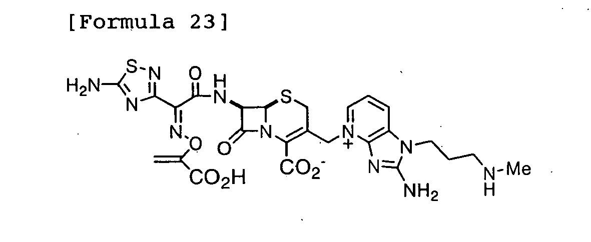

¹H-NMR (D₂O) d: 2.15 - 2.25 (2H, m), 2.70 (3H, s), 3.09 - 3.15 (2H, m), 3.21 and 3.48 (2H, ABq, J = 17.9 Hz), 5.16 (1H, d, J = 4.8 Hz), 5.20 - 5.25 (2H, m), 5.37 (1H, d, J = 2.1 Hz), 5.56 (1H, d, J = 14.7 Hz), 5.84 (1H, d, J = 4.8 Hz), 7.27 (1H, t-like), 7.87 (1H, d, J = 7.2 Hz), 8.10 (1H, d, J = 6.6 Hz).
IR (KBr) cm⁻¹: 3375, 3179, 1769, 1650, 1601, 1565, 1528, 1494, 1471, 1397, 1355, 1320, 1200, 1167, 1113, 1067, 1022.
MS(ESI): 658⁺ (M+H)⁺

| Elementary Analysis C₂₅H₂₇N₁₁O₇S₂·3.2 H₂O | | | | |
|---|---|---|---|---|
| Calc.: | C,41.98 ; | H,4.71 ; | N,21.54 ; | S,8.97 (%) |
| Found: | C,42.19 ; | H,4.84 ; | N,21.24 ; | S,8.97 (%) |

### [Example 16]

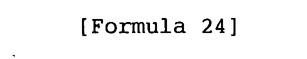

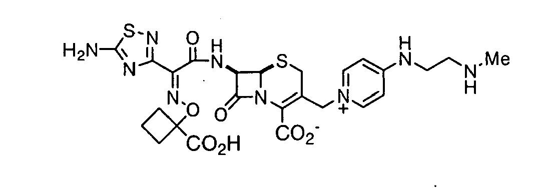

¹H-NMR (D₂O) d: 1.82 - 2.03 (2H, m), 2. 26 - 2.58 (4H, m), 2.76 (3H,s), 3.17 and 3.60 (2H, ABq, J = 18.0 Hz), 3.32 (2H, t, J = 6.0 Hz), 3.71 (2H, t, J = 6.0 Hz), 5.11 (1H, d, J = 14.7 Hz), 5.27 (1H, d, J = 4.8 Hz), 5.82 (1H, d, J = 4.8 Hz), 6.88 (2H, d, J = 7.2 Hz), 8.19 (2H, brd-like).
IR (KBr) cm⁻¹: 3377, 1770, 1666, 1602, 1566, 1494, 1471, 1397, 1319, 1233, 1162, 1145, 1067, 1022.
MS(ESI): 686⁺ (M+H)⁺

| Elementary Analysis C₂₅H₂₉N₉O₇S₂·2.4 H₂O | | | | |
|---|---|---|---|---|
| Calc.: | C,44.49 ; | H,5.05 ; | N,18.68 ; | S,9.50 (%) |
| Found: | C,44.42 ; | H,4.96 ; | N,18.78 ; | S,9.34 (%) |

### [Example 17]

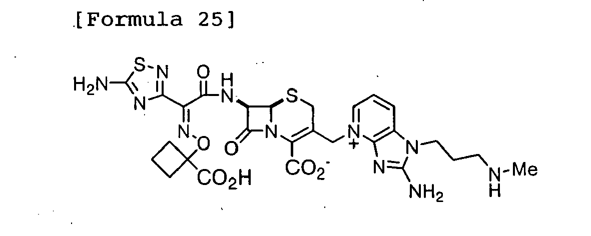

¹H-NMR (D₂O) d: 1.81 - 2.02 (2H, m), 2.15 - 2.40 (4H, m), 2.45 - 2.54 (2H, m), 2.70 (3H, s), 3.10 - 3.15 (2H, m), 3.25 and 3.52 (2H, ABq, J = 18.0 Hz), 4.22 (2H, t, J = 7.2 Hz), 5.22 (1H, d, J =4.5 Hz), 5.25 and 5.56 (2H, ABq, J = 14.4 Hz), 5.84 (1H, d, J = 4.5 Hz), 7.28 (1H, t-like), 7.87 (1H, d, J = 7.2 Hz), 8.11 (1H, d, J = 6.3 Hz).
IR (KBr) cm⁻¹: 3377, 1770, 1666, 1602, 1566, 1494, 1471, 1397, 1319, 1233, 1162, 1145, 1067, 1022.
MS(ESI) : 686⁺ (M+H)⁺

| Elementary Analysis C₂₇H₃₁N₁₁O₇S₂·3.3 H₂O | | | | |
|---|---|---|---|---|
| Calc.: | C,43.52 ; | H,5.09 ; | N,20.68 ; | S,8.61 (%) |
| Found: | C,43.49 ; | H,5.06 ; | N,20.66 ; | S,8.68 (%) |

### [Example 18]

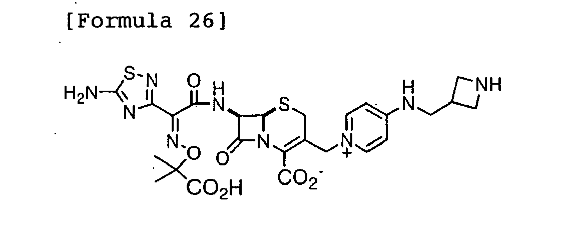

¹H-NMR (D₂O) d: 1.50 (3H, s), 1.50 (3H, s), 3.17 and 3.58 (2H, ABq, J = 18.3 Hz), 3.24 - 3.37 (1H, m), 3.62 (2H, d, J = 7.4 Hz), 3.95 (2H, dd, J = 7.4, 11.7 Hz), 4.19 - 4.25 (2H, m), 4.84 and 5.07 (2H, ABq, J = 15.3 Hz), 5.25 (1H, d, J = 4.8 Hz), 5.83 (1H, d, J = 4.8 Hz), 6.85 (2H, d, J = 7.2 Hz), 8.06 (1H, brs), 8.20 (1H, brs).
IR (KBr) cm⁻¹: 3395, 3066, 2986, 1771, 1650, 1598, 1557, 1523, 1467, 1398, 1362, 1290, 1216, 1165, 1065.
MS(ESI): 632⁺ (M+H)⁺

| Elementary Analysis C₂₅H₂₉N₉O₇S₂·3.6 H₂O | | | | |
|---|---|---|---|---|
| Calc.: | C,43.11 ; | H,5.24 ; | N,18.10 ; | S,9.21 (%) |
| Found: | C,43.04 ; | H,5.04 ; | N,18.37 ; | S,8.92 (%) |

### [Example 19]

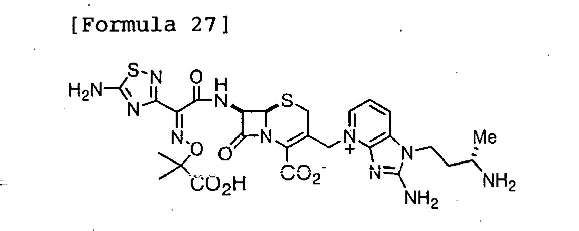

¹H-NMR (D₂O) d: 1.41 (3H, d, J = 6.6 Hz), 1.49 (3H, s), 1.50 (3H, s), 2.09 - 2.11 (1H, m), 2.15 - 2.27 (1H, m), 3.25 (1H, d, J = 17.7 Hz), 3.41 - 3.54 (2H, m), 3.24 (2H, t, J = 8.1 Hz), 5.20 (1H, d, J = 4.8 Hz), 5.25 and 5.57 (2H, ABq, J = 14.7 Hz), 5.83 (1H, d, J = 4.8 Hz), 7.30 (1H, t-like), 7.89 (1H, d, J = 7.8), 8.12 (1H, d, J = 6.6).
IR (KBr) cm⁻¹: 3368, 3180, 1770, 1651, 1566, 1495, 1469, 1399, 1362, 1320, 1222, 1162, 1066.
MS(ESI): 674⁺ (M+H)⁺

| Elementary Analysis C₂₆H₃₁N₁₁O₇S₂·3.1 H₂O | | | | |
|---|---|---|---|---|
| Calc.: | C,42.80 ; | H,5.14 ; | N,21.12 ; | S,8.79 (%) |
| Found: | C,42.96 ; | H,5.26 ; | N,20.93 ; | S,8.35 (%) |

### [Example 20]

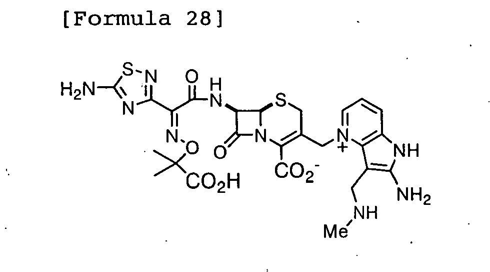

¹H-NMR (D₂O) d: 1.50 (3H, s), 1.51 (3H, s), 2.78 (3H, s), 2.97 and 3.22 (2H, ABq, J = 18.0 Hz), 4.32 and 4.53 (2H, ABq, J = 15.6 Hz),5.24 (1H, d, J = 4.8 Hz), 5.43 and 5.61 (2H, ABq, J = 15.6 Hz), 5.83 (1H, d, J = 4.8 Hz), 7.12 (1H, dd, J = 6.0, 7.5 Hz), 7.74 (1H, d, J = 7.5 Hz), 7.87 (1H, d, J = 6.0 Hz).
IR (KBr) cm⁻¹: 3381, 3189, 1768, 1577, 1526, 1469, 1399, 1362, 1284, 1227, 1158, 1066.
MS(ESI): 645⁺ (M+H)⁺

| Elementary Analysis C₂₅H₂₈N₁₀O₇S₂·5.2 H₂O | | | | |
|---|---|---|---|---|
| Calc.: | C,40.67 ; | H,5.24 ; | N,18.97 ; | S,8.69 (%) |
| Found: | C,40.57 ; | H,4.76 ; | N,18.65 ; | S,9.12 (%) |

### [Example 21]

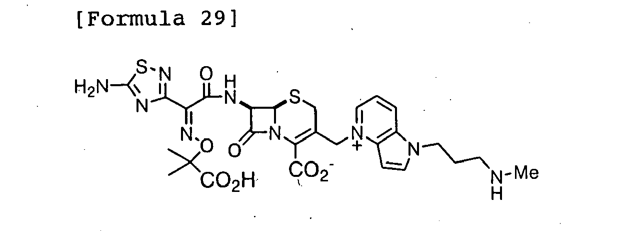

¹H-NMR (D₂O) d: 1.48 (6H, s), 2.31 (2H, m), 2.69 (3H, s), 3.03 - 3.09 (2H, m), 3.17 and 3.38 (2H, ABq, J = 17.7 Hz), 4.53 (2H, t, J = 7.2 Hz), 5.19 (1H, d, J = 4.8 Hz), 5.55 and 5.69 (2H, ABq, J = 15.0 Hz), 5.83 (1H, d, J = 4.8 Hz), 7.03 (1H, d, J = 3.8 Hz), 7.68 (1H, dd, J = 6.0, 8.1 Hz), 8.12 (1H, d, J = 3.8 Hz), 8.59 (1H, d, J = 8.1 Hz), 8.64 (1H, d, J = 6.0 Hz).
IR (KBr) cm⁻¹: 3420, 2985, 1774, 1611, 1525, 1498, 1467, 1393, 1361, 1286, 1158, 1122, 1062.
MS(ESI): 658⁺ (M+H)⁺

| Elementary Analysis C₂₇H₃₁N₉O₇S₂·5.3 H₂O | | | | |
|---|---|---|---|---|
| Calc.: | C,43.05 ; | H,5.57 ; | N,16.74 ; | S,8.51 (%) |
| Found: | C,42.98 ; | H,5.37 ; | N,16.69 ; | S,8.59 (%) |

### Experimental example 1

Antibacterial activity of Compound (I) was examined. (Test method)
Minimal inhibitory concentration (MIC: µg/ml) was determined by an agar plate dilution method using a sensitive disc mdium as a test medium with an innoculation amount of 1000 cfu/spot in conformance with the standard method established by Japanese Society of Chemotherapy.

**[Table 1]**

| (MIC: pg/ml) | | | | | | |
|---|---|---|---|---|---|---|
| Example | 1 | 2 | 5 | 10 | 12 | 19 |
| S.pneumoniae Type I | 0.125 | 0.25 | 0.125 | 0.125 | 0.25 | 0.125 |
| E.coli NIHJ JC-2 | 0.125 | 0.125 | 0.125 | 0.25 | 0.125 | 0.125 |
| P.aeruginosa SR24 | 0.5 | 0.5 | 0.5 | 0.5 | 0.25 | 0.5 |
| H.influenzae ATCC49766 | 0.063 | 0.032 | 0.063 | 0.016 | 0.032 | 0.032 |

Compounds of the present invention exhibited potent antibacterial activities against Gram-positive bacteria and Gram-negative bacteria.

### Formulation example 1

Powder of the inventive compound of Example 1 is packed to prepare an injectable agent.

## Claims

1. A compound of Formula 1: 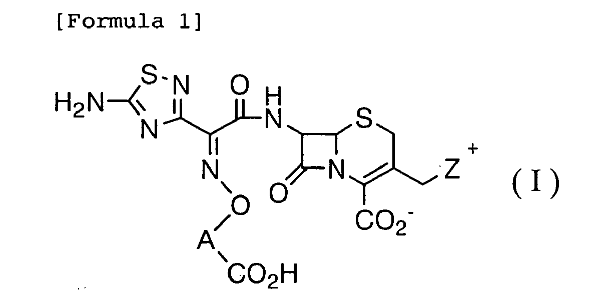 (wherein A is optionally substituted lower alkylene (substituent: mono- or di- lower alkyl, lower alkylidene, or lower alkylene having two or more carbons); Z⁺ is either of the groups shown below: 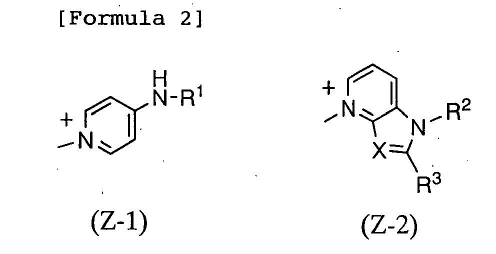 (wherein R¹ and R² are each independently hydrogen, optionally substituted amino lower alkyl or optionally substituted cyclic amino; R³ is hydrogen or amino; X is N or CR⁴ (R⁴ is hydrogen or optionally substituted lower alkyl)), a pharmaceutically acceptable salt or a solvate thereof.

2. A compound according to claim 1, wherein A is a group of Formula 3: 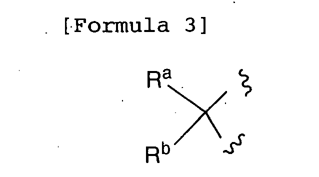 (wherein R^{a} and R^{b} are each independently hydrogen or lower alkyl, or taken together form lower alkylidene or lower alkylene having two or more carbons), a pharmaceutically acceptable salt or a solvate thereof.

3. A compound according to claim 1, wherein A is -C(CH₃)₂-, a pharmaceutically acceptable salt or a solvate thereof.

4. A compound according to claim 1 , wherein R¹ and R² are each independently hydrogen, amino lower alkyl which may be substituted with lower alkyl, or saturated 4- to 6-membered cyclic amino which may be substituted with lower alkyl; R³ is hydrogen or amino; and X is N or CR⁴(R⁴ is hydrogen, lower alkyl, or lower alkyl substituted with amino which may be substituted with lower alkyl), a pharmaceutically acceptable salt or a solvate thereof.

5. A compound according to claim 1, wherein R¹ and R² are each independently amino lower alkyl which may be substituted with lower alkyl, or saturated 4- to 6-membered cyclic amino which may be substituted with lower alkyl; R³ is amino; and X is N or CH, a pharmaceutically acceptable salt or a solvate thereof.

6. A compound according to claim 1, wherein A is a group of Formula: -C(CH₃)₂-; R¹ and R² are each independently amino lower alkyl which may be substituted with lower alkyl or saturated 4- to 6-membered cyclic amino which may be substituted with lower alkyl; R³ is hydrogen or amino; and X is N or CR⁴(R⁴ is hydrogen, lower alkyl, or lower alkyl substituted with amino which may be substituted with lower alkyl), a pharmaceutically acceptable salt or a solvate thereof.

7. A compound according to claim 1, wherein Z⁺ is a group of (Z-1); and R¹ is amino lower alkyl which may be substituted with lower alkyl, a pharmaceutically acceptable salt or a solvate thereof.

8. A compound according to claim 1, wherein A is a group of Formula: -C(CH₃)₂-; Z⁺ is a group of (Z-1); and R¹ is lower alkyl substituted with amino which may be substituted with lower alkyl, a pharmaceutically acceptable salt or a solvate thereof.

9. A compound according to claim 1, wherein A is a group of Formula: -C(CH₃)₂- ; Z⁺ is a group of (Z-1); and R¹ is -(CH₂)mNHCH₃ (m is an integer of 1 to 5), a pharmaceutically acceptable salt or a solvate thereof.

10. A compound according to claim 1, wherein A is a group of Formula: -C(CH₃)₂-; Z⁺ is a group of (Z-1); and R¹ is -(CH₂)₂NHCH₃, a pharmaceutically acceptable salt or a solvate thereof.

11. A compound according to claim 1, wherein A is a group of Formula: -C(CH₃)₂-; Z⁺ is a group of (Z-1); and R¹ is saturated 4- to 6-membered cyclic amino which may be substituted with lower alkyl, a pharmaceutically acceptable salt or a solvate thereof.

12. A compound according to claim 1, wherein Z⁺ is a group of (Z-2); and R³ is amino, a pharmaceutically acceptable salt or a solvate thereof.

13. A compound according to claim 1, wherein A is a group of Formula: -C(CH₃)₂-; Z⁺ is a group of (Z-2); and R³ is amino, a pharmaceutically acceptable salt or a solvate thereof.

14. A compound according to claim 1, wherein A is a group of Formula: -C(CH₃)₂-; Z⁺ is a group of (Z-2); and R² is hydrogen, -(CH₂)nNHCH₃ (n is an integer of 1 to 5), -(CH₂)pCH(CH₃)NH₂ (p is an integer of 1 to 5) or either one of the groups shown below: 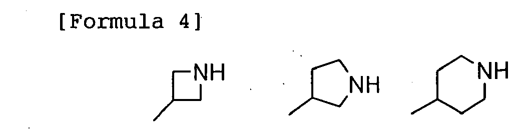 ; R³ is hydrogen or amino; and X is N or CR⁴(R⁴ is hydrogen, lower alkyl or lower alkyl substituted with amino which may be substituted with lower alkyl), a pharmaceutically acceptable salt or a solvate thereof.

15. A compound according to claim 1, wherein A is a group of Formula: -C(CH₃)₂-; Z⁺ is a group of (Z-2); R² is -(CH₂)nNHCH₃ (n is an integer of 1 to 5); R³ is amino; and X is N, a pharmaceutically acceptable salt or a solvate thereof.

16. A compound according to claim 1, wherein A is a group of Formula: -C(CH₃)₂-; Z⁺ is a group of (Z-2); R² is -(CH₂)₃NHCH₃; R³ is amino; and X is N, a pharmaceutically acceptable salt or a solvate thereof.

17. A compound according to claim 1, wherein A is a group of Formula: -C(CH₃)₂-; Z⁺ is a group of (Z-2); R² is saturated 4- to 6-membered cyclic amino which may be substituted with lower alkyl; R³ is hydrogen or amino; and X is N or CR⁴(R⁴ is hydrogen, or lower alkyl), a pharmaceutically acceptable salt or a solvate thereof.

18. A compound according to claim 1, wherein A is a group of Formula: -C(CH₃)₂-; Z⁺ is a group of (Z-2); R² is saturated 4- to 6-membered cyclic amino which may be substituted with lower alkyl; R³ is amino; and X is N or CH, a pharmaceutically acceptable salt or a solvate thereof.

19. A compound according to claim 1, wherein A is a group of Formula: -C(CH₃)₂-; Z⁺ is a group of (Z-2); R² is hydrogen; R³ is hydrogen or amino; and X is CR⁴ (R⁴ is lower alkyl or lower alkyl substituted with amino which may be substituted with lower alkyl), a pharmaceutically acceptable salt or a solvate thereof.

20. A pharmaceutical composition containing a compound according to any one of claims 1 to 19, a pharmaceutically acceptable salt or a solvate thereof.

21. A pharmaceutical composition of claim 20, which is an antibacterial agent.

22. A carboxy and/or amino-protected compound of a compound of any one of claims 1 to 19, a pharmaceutically acceptable salt or a solvate thereof.
